# EUROPEAN PATENT SPECIFICATION

(11) **EP 4 463 233 B1**
(45) Date of publication and mention of the grant of the patent: **16.04.2025**
(21) Application number: 23717262.2
(22) Date of filing: 13.03.2023
(51) Int. Cl.: A61Q 1/06, A61Q 1/02, A61Q 5/02, A61K 8/19

(54) **USE OF HUNTITE MINERAL AS A COLOR ENHANCER IN COSMETIC APPLICATIONS AND COSMETIC COMPOSITIONS CONTAINING IT**
VERWENDUNG VON HUNTITMINERAL ALS FARBVERSTÄRKER IN KOSMETISCHEN ANWENDUNGEN UND KOSMETISCHE ZUSAMMENSETZUNGEN DAMIT
UTILISATION DE MINÉRAL DE HUNTITE EN TANT QU'ACTIVATEUR DE COULEUR DANS DES APPLICATIONS COSMÉTIQUES ET COMPOSITIONS COSMÉTIQUES LE CONTENANT

(30) Priority: 11.03.2022 IT 202200004724
(43) Date of publication of application: 20.11.2024
(73) Proprietor: Prodotti Gianni S.r.l., 20138 Milano (IT)
(72) Inventor: BONDURRI, Luigi, 20138 Milano (IT); CAVENAGHI, Andrea, 20138 Milano (IT); FOGLIA, Elena, 20138 Milano (IT); GUIDOLIN, Viviana, 20138 Milano (IT)
(74) Representative: Banchetti, Marina
(86) International application number: PCT/IT2023/050077
(87) International publication number: WO 2023/170723

(56) References cited:
- WO-A1-2010/051458
- US-A1- 2009 029 902
- BARBIERI M. ET AL: "HUNTITE, A MINERAL USED IN ANTIQUITY", ARCHAEOMETRY, vol. 16, no. 2, 1 July 1974 (1974-07-01), GB, pages 211 - 220, XP093121490, ISSN: 0003-813X, Retrieved from the Internet <URL:https://dx.doi.org/10.1111/j.1475-4754.1974.tb00106.x> [retrieved on 20240122], DOI: 10.1111/j.1475-4754.1974.tb00106.x

## Description

### Field of the invention

The present invention concerns the use of the huntite mineral as a color enhancer in cosmetic applications, as well as cosmetic compositions containing it. More specifically, the invention relates to the use of huntite powder, a natural mineral of calcium and magnesium carbonate having the formula Mg₃Ca(CO₃)₄, as a color enhancer for pigments and, more generally, as a multifunctional cosmetic ingredient which, due to its morphological and optical properties, can perform various functions in cosmetics, replacing all or part of some raw materials considered harmful to the environment or which have proved to be unsafe for human health. In the cosmetic compositions in which it is inserted, huntite also improves some functional and technological characteristics.

### Background of the invention

Among the raw materials of the cosmetic industry, those in powder form represent a very important and varied family of ingredients, being used in many cosmetic products both in emulsion, in gel and in the form of solid products. Among the latter there are the anhydrous, essentially formed by oils and waxes, which once melted are poured into stick molds (like lipsticks) or in jars, and the powders, which can be left free, or can be compacted using presses, or cooked in specific ovens to obtain the common wafers.

Raw materials in powder form are used in many cosmetic products, especially for make-up, and can have various functions, such as for example the functions of texturizers, fillers, absorbents, mattifying agents, "soft-focus" enhancers and others.

**Fillers** are used in powder formulations with the main purpose of diluting the active components and lowering the cost of the formulation. The most common and used filler powder is talc, which is inexpensive and extremely smooth and soft to the touch; however, the cosmetic market is gradually moving away from this ingredient, due to the risk of possible asbestos contamination that this raw material presents and, therefore, the risk of probable consequences on health >Gordon R.E. et al., Asbestos in commercial cosmetic talcum powder as a cause of mesothelioma in women, IJOEH 2014, 20(4): 318-332; Tran, T.H. et al., Talc, Asbestos, and Epidemiology: Corporate Influence and Scientific Incognizance, Epidemiology 2019, 30 (6): 783-788).

Other powders have been considered as substitutes for talc: an example is mica, which however has a higher price, is greyish and does not have the soft touch of talc. Other alternatives are kaolin, which however absorbs sebum a lot and risks excessively drying the skin, or vegetable starches or clays, which however very often present problems linked to microbiological contamination.

Coloring powders, more commonly referred to as **pigments,** have the characteristic of releasing color when applied to a surface and therefore hiding the color of the surface itself: they therefore have the ability to "write". The "writing ability" is an intrinsic characteristic of some powders and is directly proportional to their refractive index: the higher the refractive index, the more difficult it is to see transparently through the powder and vice versa. The most used pigments in cosmetics are ionic compounds, or metal oxides, and among the most common are iron oxides (yellow, red, black), zinc oxide (white) and titanium dioxide (white).

The latter is undoubtedly the most widely used pigment, firstly because white remains the base color on which to prepare all the other colors, and secondly because it has a coverage and a writing effect superior to all the other whites on the market. However, the use of titanium dioxide involves the fact that this compound has been classified as a carcinogen by inhalation as a non-negligible problem, which implies possible risks on the safety of cosmetic products that contain it, especially when it comes to make-up powders. -up, easily inhalable (Baan, R.A., Carcinogenic Hazards from Inhaled Carbon Black, Titanium Dioxide, and Talc not Containing Asbestos or Asbestiform Fibers: Recent Evaluations by an IARC Monographs Working Group, Inhal. Toxicol. 2007, 19: 213-228).

Another type of powder commonly used in cosmetic technology are **texturizers** (also called, in English, "skin-feel enhancers"), i.e. those ingredients that have the purpose of improving the touch of cosmetic products, making them more velvety, creamy, smooth when picked up and/or applied. In general, these properties largely depend on the size and shape of the particles that compose them, on the coefficient of friction between particles and on their elasticity. Most of these powders are composed of synthetic, non-biodegradable polymers, commonly called "microplastics" (nylon, PMMA, PTFE, polyurethanes, etc.), whose main problem is that they are not sustainable and irreversibly pollute the environment (Gouin T., et al., A Thermodynamic Approach for Assessing the Environmental Exposure of Chemicals Absorbed to Microplastic, Environ. Sci. Technol. 2011, 45(4): 1466-1472). Other solid powder materials used as texturizers, but more expensive, are silica powder, characterized by perfectly spherical particles, and mica itself, which is composed of lamellar particles.

Some powders are specifically chosen for their **"soft-focus" effect,** i.e. the ability to enter the hollows of the skin and reflect light in different directions in order to hide wrinkles and imperfections, mattifying the complexion and making it more homogeneous. Sometimes this characteristic is also combined with the absorption capacity of oils, which allows both to obtain less greasy formulas and to absorb excess sebum, eliminating the shiny effect of the complexion.

It is evident from the foregoing that it would be extremely useful to have a raw material in powder form that can be used in the cosmetic industry to improve the performance of the ingredients mentioned above, or as a total or partial replacement of those ingredients that have proved to be potentially harmful to the environment and/or hazardous to human health, such as microplastics, talc and titanium dioxide in particular. In particular, it would be extremely advantageous to be able to include in the composition of a cosmetic make-up product a multifunctional ingredient which allows to enhance the color of the pigments and to replace at least partially the titanium dioxide as a white pigment.

US 2009/029902 A1 discloses the use of calcium carbonate materials such as huntite powder as a multifunctional ingredient for the preparation of various compositions, including cosmetic products for make-up.

### Summary of the invention

In the frame of the studies which led to the present invention, a mineral of calcium and magnesium carbonate was taken into consideration, huntite, which had been studied and used in recent decades due to the excellent properties of its natural mixture with hydromagnesite as a flame retardant (Kangal O., et al., A new industrial mineral: Huntite and its recovery, Minerals Enginering 2006, 19, 376-378; Hollingbery, L.A., The Fire Retardant Behavior of Huntite and Hydromagnesite - A Review, Polym. Degr. Stab. 2010, 95, 2213-2225).

Huntite is a mineral composed of calcium and magnesium carbonates, having the chemical formula Mg₃Ca(CO₃)₄, which crystallizes in trigonal systems and is typically found in deposits in dusty masses characterized by planar crystals wet with water. Once extracted, dried and broken up, its form is that of a very fine and airy white lamellar powder which does not pack or agglomerate. By way of illustration, **Figure 1** of the accompanying illustrations shows a photomicrograph of huntite crystals (on the right) flanked by hydromagnesite crystals (on the left), while **Figure 2** shows the three-dimensional structure of the huntite crystal lattice and **Figure 3** is a photograph of a huntite powder obtained from Greek ore.

It is also known that huntite, due to its lamellar structure and its white color, can be used in paints as a filler powder (extender), thus allowing to reduce the amount of pigment (for example, TiO₂) thanks to its disruptive and light diffusing properties (Sibelco, https://coatings.sibelcotools.com/mineral/huntite/).

Furthermore, mixed carbonate materials of magnesium and calcium of formula Mg₃Ca(CO₃)₄ corresponding to huntite can also be synthesized in the laboratory through the use of carbonates as reagents, or through some strains of cyanobacteria.

According to the present invention, it has been found that huntite possesses physico-chemical and morphological properties such as to make it suitable for carrying out various specific functions in a cosmetic composition, according to the formulation in which it is inserted. These properties enable this material to replace, in whole or in part, raw materials in powder form such as talc, microplastics or titanium dioxide, the use of which in a cosmetic product is not recommended for reasons of environmental sustainability, of health risks or commercial opportunity.

In particular, it has been found that huntite powder, inserted in a suitable quantity in make-up formulations such as lipsticks, eye shadows, foundations, concealers and nail polishes, has the ability to enhance the color of the pigments contained in these formulations, increasing its intensity and brilliance. This also makes it possible to reduce the content of colored ingredients, such as pigments and lacquers, obtaining an equivalent or superior final result.

Therefore, the present invention proposes to use huntite powder as a color enhancer for pigments, and in general as a multifunctional cosmetic ingredient, exploiting its main characteristics related to the lamellar shape of its powder and the complete absence of agglomerates, which make it very soft and airy to the touch, easily workable and dispersible in liquids. Huntite generally has a very high white point according to ISO2470 (above 95%), a refractive index ranging from 1.615 to 1.625 and a medium oil absorption degree, ranging from 50 to 60 g/100 g according to method ISO 787/5.

It should be noted that in addition to presenting the properties already known and exploited in the paint sector, such as the white color and the disintegrating capacity with respect to other powders due to its lamellar structure, powdered huntite proves to be unexpectedly advantageous as a powder material the production of cosmetic preparations because it also has a soft touch, unlike, for example, mica, as well as an adhesive capacity towards the skin. If used in cosmetic products, it gives them various additional effects in addition to increasing the writing ability of the other pigments, such as improving the texture of the product and the soft-focus effect.

### Brief description of the drawings

The specific features of the invention, as well as its advantages, will become more evident with reference to the attached figure, wherein:

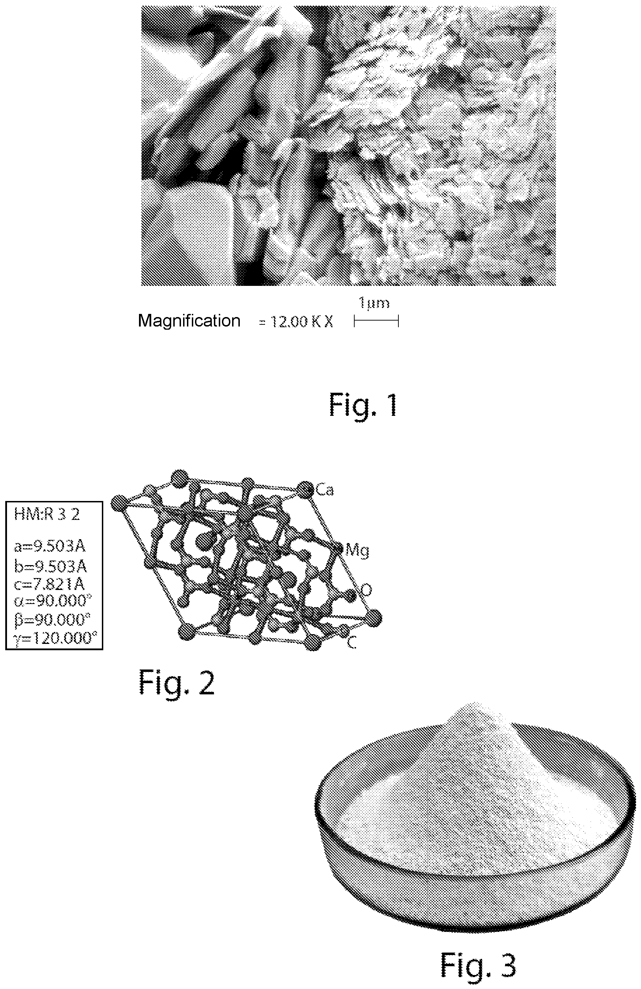

**Figure 1****,** already mentioned, shows a photomicrograph of huntite crystals (on the right), together with hydromagnesite crystals (on the left);
**Figure 2****,** also already mentioned, shows the three-dimensional structure of the huntite crystal lattice, with cell parameters;
**Figure 3****,** also already mentioned, shows a photograph of a huntite powder usable for the purposes of the present invention;

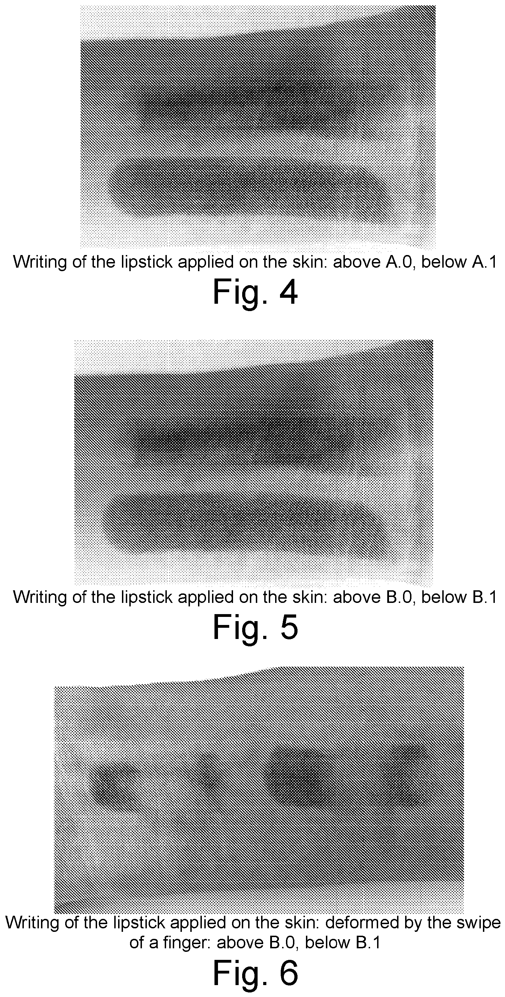

**Figure 4** shows the "writing" of A.1, a lipstick containing the ingredient according to the invention (powdered huntite, compared to A.0, a lipstick of similar composition which does not contain it;
**Figure 5** shows the "writing" of B.1, a lipstick containing powdered huntite compared to B.0, a lipstick of similar composition which instead contains an equal quantity of mica;
**Figure 6** shows the "writings" of the same lipsticks, B.0 and B.1, of the previous figure, deformed by the passage of a finger;

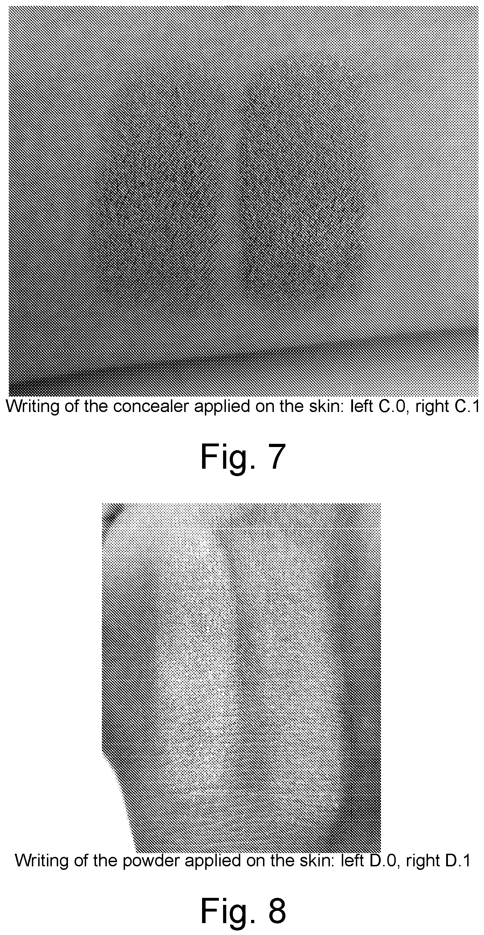

**Figure 7** shows the "writing" of C.1, a concealer containing powdered huntite compared to C.0, a concealer of similar composition which instead contains an equal amount of mica;
**Figure 8** shows the "writing" of D.1. a compact powder containing powdered huntite compared to D.0, a compact powder of similar composition which instead contains an equal amount of titanium dioxide;

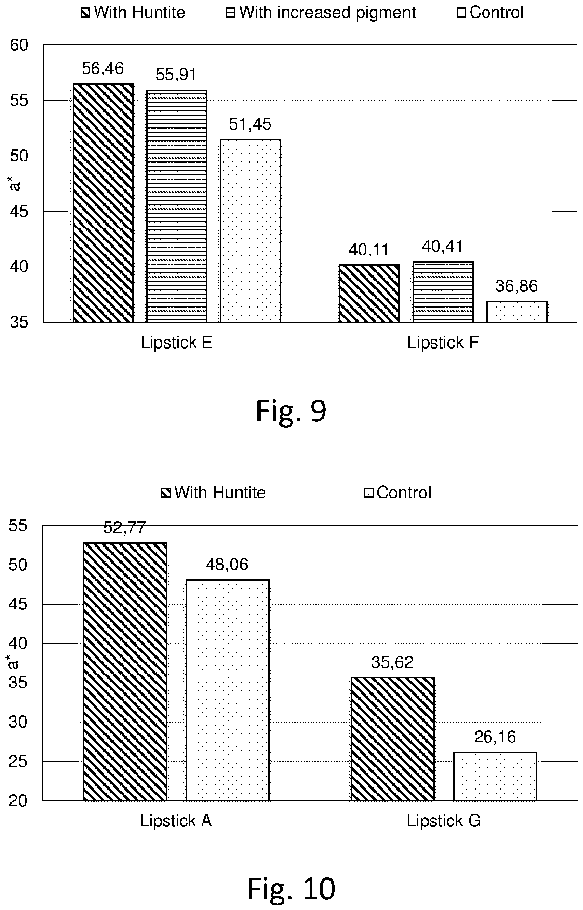

**Figure 9** is a diagram showing the results of an instrumental comparison test for the evaluation of the color yield of the solid lipstick formulations of Examples 6 and 7, in which E.1 and F.1 represent the formulations according to the invention;
**Figure 10** is a diagram showing the results of an instrumental comparison test for the evaluation of the color yield of the solid lipstick formulations of Examples 1 and 8, in which A.1 and G.1 represent the formulations according to the invention; and

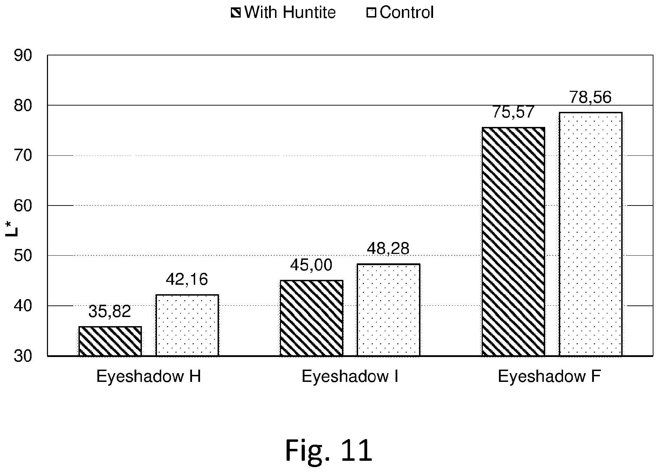

**Figure 11** is a diagram showing the results of an instrumental comparison test for evaluating the color yield of the eyeshadow formulations of Examples 9, 10 and 11, in which H.1. I.1 and L.1 represent the formulations according to the invention.

### Detailed description of the invention

The present invention specifically provides the use of natural huntite powder as a color enhancer for pigments, with multiple possible additional functions, in cosmetic products for make-up as defined by claim 1 of the appended claims.

As a color release enhancer in make-up formulations, huntite has proved to be particularly advantageous, as the fine and lamellar shape of the particles allows them to act as a disintegrant by breaking up the agglomerates of pigment during processing. In this way, the yield of the color is significantly improved when considering the writing ability, and the color is more opaque, uniform and brilliant. This property allows the formulation of make-up products (lipsticks, foundations, eye shadows, nail polishes, etc.) with a lower percentage of pigment, and facilitates their processing during preparation.

Furthermore, huntite has a very high white point, even if the refractive index is low compared to TiO₂; the fact that it is very fine and lamellar allows a homogeneous distribution on the skin and consequently the opacity obtained is comparable to that of titanium dioxide. If the powder is wetted in water or oil, it loses this characteristic and the opacity is reduced because the particles are distributed in the liquid and no longer adhere to the surface. This feature allows a formulator to design powder products for make-up, such as free, compact and cooked powders, without the use of other white pigments.

It should be noted that the other properties of the powdered huntite illustrated in the summary of the present invention are also much sought after in a cosmetic powder, because they guarantee various further improvements. Specifically, huntite can have the following additional uses and applications in the cosmetic sector:
- As an mattifying agent in transparent gels or tensiolites: although when wet it no longer possesses the same writing properties, huntite powder dispersed in a liquid maintains strong scattering, i.e. it scatters light inside the liquid making it opaque; moreover, being lamellar and fine, it remains well dispersed and does not re-precipitate, allowing the formulation of translucent or completely opaque gels, shampoos or tensiolites in general without the use of pigments or acrylic mattifying agents.
- The same concept can be applied in sunscreen formulations containing inorganic filters. Being the inorganic filters of metal oxides (titanium oxide and zinc dioxide), they are often found in agglomerated form; the addition of huntite during the processing of the product makes the dispersion of the filters more homogeneous, allowing its use in a lower percentage and overall improving the sensoriality of the formulation.
- As an adhesion enhancer: the thin and lamellar shape allows for greater adhesion to the skin, due to the friction that the lamellae make when sliding between them or on the skin. The huntite therefore reduces smudges, thus allowing a long-lasting effect.
- As a texturizer: the fine but airy shape is characterized by a soft touch, which takes the form of a velvety "skin-feel" when applied, the formulas containing huntite are less rough and creamier when applied; once absorbed by the skin, a pleasant velvety sensation remains, given by the powder that remains adhered to the skin.
- For the soft-focus effect: the fine powder is deposited in wrinkles and unifies the complexion and, moreover, remains well adhered without accumulating in the skin folds.

Therefore, according to a preferred embodiment of the present invention, huntite for cosmetic use, in addition to the function of color enhancer, has one or more of the following additional functions: texturizing agent, filler, mattifying agent, dispersant, adhesion enhancer, soft-focus effect enhancer. In view of the foregoing, the concerned huntite can be used in many types of cosmetic products: from powders, to casts, to emulsions, to tensiolites, preferably as a total or partial replacement of ingredients such as titanium dioxide, talc, mica or microplastics, whose presence in a cosmetic formulation is unwanted for safety, sustainability and/or cost reasons. In such products, huntite performs the same functions as the ingredients it replaces, while also bringing additional benefits, due to its multiple functionality.

For the cosmetic applications of the invention, the huntite powder has particle sizes between 0.1 µm and 50 µm, preferably between 0.5 µm and 10 µm, or in some cases it is entirely submicronic, meaning that 97% of the particles have a size of less than 0.90 µm.

According to a further aspect thereof, the present invention relates to a cosmetic composition for make-up, which is characterized in that it contains one or more pigments, and natural huntite powder as color enhancer, as defined by claim 6 of the appended claims.

More specifically, the cosmetic product made with the composition is a lipstick or a concealer, and the composition may preferably comprise from 3% to 12% by weight of powdered huntite powder as a color enhancer, having additional functions as texturizer and adhesion enhancer.

In further embodiments, the cosmetic product made with the composition is a nail polish, and the composition of the product may comprise 2% to 15% by weight of powdered huntite as a color enhancer.

In further embodiments, the product made with the composition is an eyeshadow, and the composition thereof may preferably comprise from 2% to 15% by weight of powdered huntite powder as a color enhancer, with additional functions of texturizer and adhesion enhancer.

In still further embodiments, the product obtained from the composition of the invention is a mattifying compact powder, and the composition preferably comprises from 20% to 30% by weight of powdered huntite as a white pigment, texturizer and soft focus agent.

Thanks to the characteristics of the raw material, huntite powder can be handled as easily as other cosmetic powders. It is easily dispersible in water or oils with common laboratory and industrial instruments, such as turboemulsifiers and mixers. For an optimal result it is possible to carry out some passages in a three-cylinder laminator together with the other powders of the formulation. Due to the nature of the mineral, it is possible to treat huntite even at high temperatures without degradation or other chemical-physical transformations.

The other ingredients used in the formulation of the cosmetic compositions according to the invention may include, in addition to the multifunctional ingredient of the present invention, all the ingredients commonly used in cosmetic formulations, such as structuring agents, emollients, pigments, texturizers, film formers, preservatives, fillers or carriers, surfactants, rheological modifiers, mattifying agents and pearlescent agents, conditioners, emulsifiers, binders, solvents, UV filters and others, where "others" are intended as optional secondary ingredients that improve aspects of the sensoriality and functionality or of the stability of the formula, for example perfumes, flavoring, gloss enhancers, active ingredients, antioxidants, chelators, stabilizers, pH regulators. These ingredients will be selected according to the current cosmetic technique, based on the desired properties in the final product.

### EXAMPLES

Some specific embodiments of the cosmetic use of huntite according to the present invention, with the corresponding products, and including some experimental results, are reported by way of not limiting examples in the following.

### Example 1

### Colored solid lipstick with dispersions of organic and inorganic pigments in di-isostearyl maleate. Formulation containing huntite compared with a formulation without huntite

Two solid lipsticks having the composition indicated below were obtained by applying the procedure reported after the table.

| **Phase** | **Ingredient (INCI name)** | **Function** | **Composition of A.0 (%)** | **Composition of A.1 (%)** |
|---|---|---|---|---|
| A | Pentaerythrityl Tetraethyl-hexanoate | Emollient | 32.00 | 27.00 |
| | Polyglyceryl-2 Isostearate | Emollient | 14.00 | 14.00 |
| | Triethylhexanoin | Emollient | 12.50 | 12.50 |
| | Tridecyl Trimellitate | Emollient, gloss enhancer | 18.00 | 18.00 |
| B | Synthetic Wax, Cera Micro-cristallina | Structuring agent | 8.00 | 8.00 |
| | Polyethylene | Structuring agent | 3.50 | 3.50 |
| | Copernicia Cerifera Wax (carnauba wax) | Structuring agent | 2.00 | 2.00 |
| C | CI 77891 (titanium dioxide), Diisostearyl Malate, Isopropyl Titanium Triisostearate | Pigment | 5.00 | 5.00 |
| | Diisostearyl Malate, CI 15850 (Red 6 lake), Isopropyl Titanium Triisostearate | Pigment | 4.80 | 4.80 |
| | Diisostearyl Malate, CI 42090 (Blue 1 lake), Isopropyl Titanium Triisostearate | Pigment | 0,20 | 0,20 |
| | Huntite | Texturizer, adhesion enhancer, color enhancer | - | 5 |
| | - | | | |

These lipsticks can be prepared by heating the phase A and phase B ingredients until the waxes are completely melted and incorporating the phase C ingredients under constant stirring. The product is then poured into a lipstick mold at a temperature of 85 °C.

As can also be seen in **Figure 4****,** the lipstick A.1 according to the invention has a more marked blue shade, a sign that the pigment has developed better; the release of the pastel is similar, but on a color level, A.1 is more opaque and the color more vivid. Even the brightness of the product is not affected by the use of huntite. Furthermore, the application of A.1 is more homogeneous and much smoother on test than the application of A.0.

It can be concluded that huntite improves color release and development, and can be effectively used as a texturizer to improve the smoothness of pastels.

A further experimentation on the properties of the lipsticks obtained is presented in the final section of the present description.

### Example 2

### Colored solid lipstick with pure organic pigments (lakes). Formulation containing huntite compared with a formulation containing sericite (mica)

Two solid lipsticks having the composition indicated below were obtained by applying the procedure reported after the table.

| **Phase** | **Ingredient (INCI name)** | **Function** | **Composition of B.0 (%)** | **Composition of B.1 (%)** |
|---|---|---|---|---|
| A | Pentaerythrityl Tetraethyl-hexanoate | Emollient | 30,00 | 30,00 |
| | Polyglyceryl-2 Isostearate | Emollient | 11,00 | 11,00 |
| | Triethylhexanoin | Emollient | 12,50 | 12,50 |
| | Tridecyl Trimellitate | Emollient, gloss enhancer | 17,00 | 17,00 |
| B | Synthetic Wax, Cera Micro-cristallina | Structuring agent | 8,00 | 8,00 |
| | Polyethylene | Structuring agent | 3,50 | 3,50 |
| | Copernicia Cerifera Wax (carnauba wax) | Structuring agent | 2,00 | 2,00 |
| C | CI 77891 (titanium dioxide), Diisostearyl Malate, Isopropyl Titanium Triisostearate | Pigment | 5,00 | 5,00 |
| | CI 15850 (Red 6 lake) | Pigment | 3,00 | 3,00 |
| | CI 15850 (Red 7 lake) | Pigment | 2,00 | 2,00 |
| | CI19140 (Yellow 5 lake) | Pigment | 1,00 | 1,00 |
| | Huntite | Texturizer, adhesion enhancer, color enhancer | - | 10,00 |
| | - | | | |
| | Mica | Texturizer, adhesion enhancer | 10,00 | - |

These lipsticks can be prepared by heating the phase A ingredients and the phase B ingredients until the waxes are completely melted. The ingredients of phase C are added to the molten mass and then the whole is rolled in a three-cylinder laminator to obtain a homogeneous paste. The product is poured into a lipstick mold at a temperature of 85 °C.

As can also be seen from **Figures 5** and **6****,** lipstick B.1 according to the invention has a more intense color when applied and is smoother than lipstick B.0. Huntite therefore improves ease, homogeneity of application and color release. There is also an increase in adhesion, i.e. a greater resistance to smudging.

It can be concluded, as previously, that huntite improves the release and development of color, and that it improves the application of the product and increases its adhesion compared to the same formulation containing sericite.

### Example 3

### Colored solid concealer with inorganic pigments. Formulation containing huntite compared with a formulation containing mica

Two solid concealers having the composition indicated below were obtained by applying the procedure reported after the table.

| **Phase** | **Ingredient (INCI name)** | **Function** | **Composition of C.0 (%)** | **Composition of C.1 (%)** |
|---|---|---|---|---|
| A | Trioctyldodecyl Citrate | Emollient | 35.60 | 35.60 |
| | Pentaerythrityl Tetra-ethylhexanoate | Emollient | 17.00 | 17.00 |
| | Triethylhexanoin | Emollient | 10.00 | 10.00 |
| B | Synthetic Wax, Cera Micro-cristallina | Structuring agent | 2.00 | 2.00 |
| | Polyethylene | Structuring agent | 4.00 | 4.00 |
| | Euphorbia cerifera Wax (candelilla wax) | Structuring agent | 3.50 | 3.50 |
| C | CI 77891 (titanium dioxide) | Pigment | 12.60 | 12.60 |
| | CI 77492 (yellow iron oxide) | Pigment | 2.00 | 2.00 |
| | CI 77491 (red iron oxide) | Pigment | 0.60 | 0.60 |
| | CI 77499 (black iron oxide) | Pigment | 0.20 | 0.20 |
| | Huntite | Texturizer, adhesion enhancer, color enhancer | - | 10.00 |
| | - | | | |
| | *Mica* | Texturizer, adhesion enhancer | 10.00 | - |
| | *Silica* | Texturizer | 2.50 | 2.50 |

These products can be prepared by heating the phase A ingredients and the phase B ingredients until the waxes are completely melted. The ingredients of phase C are added to the molten mass and then the whole is rolled in a three-cylinder laminator to obtain a homogeneous paste. The product is poured into an appropriate casing at a temperature of 85 °C.

It has been found that the C.1 version of the concealer according to the invention is more easily workable, because the huntite does not increase the viscosity of the product, which instead happens in the version with mica. As can also be seen in **Figure 7****,** produced with huntite does not create color halos, has a more homogeneous application, a more vivid and full color and has a better wrinkle filling effect than sample C.0.

Also from a sensory point of view, the C.1 version is softer to apply and easier to apply.

### Example 4

### Compact mattifying powder. Formulation containing huntite compared with a formulation containing titanium dioxide

Two compact mattifying powders having the composition indicated below were obtained by applying the procedure reported after the table.

| **Phase** | **Ingredient (INCI name)** | **Function** | **Composition of D.0 (%)** | **Composition of D.1 (%)** |
|---|---|---|---|---|
| A | Mica | Filler | 52.40 | 52.40 |
| | Magnesium myristate | Structuring agent | 2.00 | 2.00 |
| | Lauroyl lisine | Texturizer | 1.50 | 1.50 |
| | Boron nitride | Texturizer | 2.50 | 2.50 |
| | Silica | Texturizer | 2.00 | 2.00 |
| B | Huntite | White pigment, texturizer, soft-focus enhancer | - | 28.20 |
| | - | | | |
| | CI 77891 (titanium dioxide) | Pigment | 28.20 | - |
| | CI 77492 (yellow iron oxide) | Pigment | 1.00 | 1.00 |
| | CI 77491 (red iron oxide) | Pigment | ..0.50 | ..0.50 |
| | CI 77499 (black iron oxide) | Pigment | | 0.30 |
| C | Isopropyl isostearate | Binder | 3.50 | 3.50 |
| | Trioctyldodecyl Citrate | Binder | 2.00 | ..2.00 |
| | Pentaerythrityl Tetraethylhexanoate | Binder | 1.80 | 1.80 |
| D | Dimethicone dimethiconol | Binder | 1.50 | 1.50 |
| | Phenoxyetanol, Caprylil glycol | Preservative | 080 | 080 |

These products are obtained by inserting the ingredients of phases A and B into a mill and mixing until a homogeneous powder is obtained. The ingredients of phases C and D are then introduced, mixing until homogeneity. The product is then compacted with a special press in a bottom.

As can also be seen from **Figure 8****,** the product D.1 according to the invention, containing huntite, is opaque, even if not as much as that formulated with titanium dioxide; it has been seen that huntite can still be used in powder formulations to obtain medium coverage products, without titanium dioxide.

Furthermore, during application the D.1 product flows more and is more homogeneous, making it easier to apply on the face. There is also an excellent wrinkle filling and soft-focus effect.

### Example 6

### Colored solid lipstick with pure organic pigments (Red 7 lake). Formulation containing huntite compared with two formulations without huntite

Three solid lipsticks having the composition indicated below were obtained by applying the procedure reported after the table.

| **Phase** | **Ingredient (INCI name)** | **Function** | **Composition of E.0 (%)** | **Composition of E.1 (%)** | **Composition of E.2 (%)** |
|---|---|---|---|---|---|
| A | Triisocetyl Citrate | Emollient | 32.41 | 29.00 | 31.56 |
| | Triethylhexanoin | Emollient | 22.35 | 20.00 | 21.76 |
| | Trioctyldodecyl Myristate | Emollient | 10.06 | 9.00 | 9.79 |
| | Cetyl Ethylhexanoate | Emollient | 11.74 | 10.50 | 11.43 |
| B | Euphorbia cerifera Wax (candelilla wax) | Structuring agent | 8.94 | 8.00 | 8.71 |
| | Helianthus Annuus Seed Oil, Hydrogenated Vegetable Oil | Structuring agent | 6.71 | 6.00 | 6.53 |
| | Myristic/Palmitic/Stearic/ Ricinoleic/Eicosanedioic Glycerides, Dextrin Palmitate | Structuring agent | 2.24 | 2.00 | 2.18 |
| C | CI 15850 (Red 7 lake) | Pigment | **5.00** | **5.00** | **7.50** |
| | Huntite | Texturizer, adhesion enhancer, esaltatore di colore | - | **10.00** | - |
| | - | | | | |
| D | Tocopherol | Antioxidant | 0.22 | 0.20 | 0.22 |
| | Aroma | Aroma | 0.34 | 0.30 | 0.33 |

These lipsticks can be prepared by heating the phase A ingredients and the phase B ingredients until the waxes are completely melted. The ingredients of phase C are added to the molten mass and then the whole is rolled in a three-cylinder laminator to obtain a homogeneous paste. The product is then heated until it is completely melted and the ingredients of phase D are added. It is then poured into a lipstick mold at a temperature of 85 °C.

Experimentation on the properties of the lipsticks obtained is presented in the final section of the present description.

### Example 7

### Solid lipstick with red iron oxide pigments. Formulation containing huntite compared with two formulations without huntite

Three solid lipsticks having the composition indicated below were obtained by applying the procedure reported after the table.

| **Phase** | **Ingredient (INCI name)** | **Function** | **Composition of F.0 (%)** | **Composition of F.1 (%)** | **Composition of F.2 (%)** |
|---|---|---|---|---|---|
| A | Triisocetyl Citrate | Emollient | 32.41 | 29.00 | 31.22 |
| | Triethylhexanoin | Emollient | 22.35 | 20.00 | 21.53 |
| | Trioctyldodecyl Myristate | Emollient | 10.06 | 9.00 | 9.69 |
| | Cetyl Ethylhexanoate | Emollient | 11.74 | 10.50 | 11.30 |
| B | Euphorbia cerifera Wax (candelilla wax) | Structuring agent | 8.94 | 8.00 | 8.61 |
| | Helianthus Annuus Seed Oil, Hydrogenated Vegetable Oil | Structuring agent | 6.71 | 6.00 | 6.46 |
| | Myristic/Palmitic/Stearic/Ricinoleic/Eicosanedi oic Glycerides, Dextrin Palmitate | Structuring agent | 2.24 | 2.00 | 2.15 |
| C | CI 77491 (red iron oxide) | Pigment | **5.00** | **5.00** | 8.50 |
| | Huntite | Texturizer, adhesion enhancer, color enhancer | - | **10.00** | - |
| | - | | | | |
| D | Tocopherol | Antioxidant | 0.22 | 0.20 | 0.22 |
| | Aroma | Aroma | 0.34 | 0.30 | 0.32 |

These lipsticks can be prepared with a procedure similar to that of Example 6.

Experimentation on the properties of the lipsticks obtained is presented in the final section of the present description.

### Example 8

### Solid pearly lipstick with red and white pearl. Formulation containing huntite compared with a formulation without huntite

Two solid lipsticks having the composition indicated below were obtained by applying the procedure reported after the table.

| **Phase** | **Ingredient (*INCI name)*** | **Function** | **Composition of G.0 (%)** | **Composition of G.1 (%)** |
|---|---|---|---|---|
| A | Triisocetyl Citrate | Emollient | 32.22 | 29.00 |
| | Triethylhexanoin | Emollient | 22.22 | 20.00 |
| | Trioctyldodecyl Myristate | Emollient | 10.00 | 9.00 |
| | Cetyl Ethylhexanoate | Emollient | 11.67 | 10.50 |
| B | Euphorbia cerifera Wax (candelilla wax) | Structuring agent | 8.89 | 8.00 |
| | Helianthus Annuus Seed Oil, Hydrogenated Vegetable Oil | Structuring agent | 6.67 | 6.00 |
| | Myristic/Palmitic/Stearic/ Ricinoleic/Eicosanedioic Glycerides, Dextrin Palmitate | Structuring agent | 2.22 | 2.00 |
| C | Mica, CI 77891 (titanium dioxide) | Pigment | 2.22 | 2.00 |
| | Mica, CI 77491 (iron oxide) | Pigment | 3.33 | 3.00 |
| | Huntite - | Texturizer, adhesion enhancer, color enhancer | - | **10.00** |
| D | Tocopherol | Antioxidant | 0.22 | 0.20 |
| | Aroma | Aroma | 0.33 | 0.30 |

These lipsticks can be prepared with a procedure similar to that of Example 6.

Experimentation on the properties of the lipsticks obtained is presented in the final section of the present description.

### Example 9

### Dark eyeshadow with red and black iron oxide pigments. Formulation containing huntite compared with a formulation without huntite

Two compact powder eyeshadows having the composition indicated below were obtained by applying the procedure reported after the table.

| **Phase** | **Ingredient (*INCI name)*** | **Function** | **Composition of H.0 (%)** | **Composition of H.1 (%)** |
|---|---|---|---|---|
| A | Synthetic Fluorphlogopite | Filler | 62.20 | 57.80 |
| | Magnesium Myristate | Texturizer | 2.00 | 2.00 |
| | Calcium Sodium Borosilicate | Texturizer | 3.50 | 3.50 |
| | Huntite - | Texturizer, adhesion enhancer, color enhancer | - | **5.00** |
| | Boron Nitride | Texturizer | 1.50 | 1.50 |
| | Zea Mays Starch, Polyvinyl Alcohol, Glycerin | Texturizer | 2.00 | 2.00 |
| B | CI 77491 (red iron oxide) | Pigment | 8.00 | 8.00 |
| | CI 77499 (black iron oxide) | Pigment | 12.00 | 12.00 |
| C | Polyglyceryl-10 Decamacadamiate | Binder | 0.70 | 0.70 |
| | Pentaerythrityl Tetraethylhexanoate | Binder | 2.50 | 2.50 |
| | Glyceryl Triacetyl Ricinoleate | Binder | 1.80 | 1.80 |
| | Cetearyl Ethylhexanoate | Binder | 2.70 | 2.70 |
| D | Phenoxyetanol, Caprylil glycol | Preservative | 0.90 | 0.90 |
| | Tocopherol | Antioxidant | 0.20 | 0.20 |

These eyeshadows can be prepared with a procedure similar to that of Example 4.

Experimentation on the properties of the eyeshadows obtained is presented in the final section of the present description.

### Example 10

### Brown eyeshadow with red, black and white iron oxide pigments. Formulation containing huntite compared with a formulation without huntite

Two compact powder eyeshadows having the composition indicated below were obtained by applying the procedure reported after the table.

| **Phase** | **Ingredient (*INCI name)*** | **Function** | **Composition of I.0 (%)** | **Composition of 1.1 (%)** |
|---|---|---|---|---|
| A | Synthetic Fluorphlogopite | Filler | 62.20 | 57.80 |
| | Magnesium Myristate | Texturizer | 2.00 | 2.00 |
| | Calcium Sodium Borosilicate | Texturizer | 3.50 | 3.50 |
| | Huntite - | Texturizer, adhesion enhancer, colo enhancer | - | **5.00** |
| | Boron Nitride | Texturizer | 1.50 | 1.50 |
| | Zea Mays Starch, Polyvinyl Alcohol, Glycerin | Texturizer | 2.00 | 2.00 |
| B | CI 77891 (titanium dioxide) | Pigment | 8.00 | 8.00 |
| | CI 77491 (red iron oxide) | Pigment | 8.00 | 8.00 |
| | CI 77499 (black iron oxide) | Pigment | 12.00 | 12.00 |
| C | Polyglyceryl-10 Decamacadamiate | Binder | 0.70 | 0.70 |
| | Pentaerythrityl Tetraethyl hexanoate | Binder | 2.50 | 2.50 |
| | Glyceryl Triacetyl Ricinoleate | Binder | 1.80 | 1.80 |
| | Cetearyl Ethylhexanoate | Binder | 2.70 | 2.70 |
| D | Phenoxyetanol, Caprylil glycol | Preservative | 0.90 | 0.90 |
| | Tocopherol | Antioxidant | 0.20 | 0.20 |

These eyeshadows can be prepared with a procedure similar to that of Example 4.

Experimentation on the properties of the eyeshadows obtained is presented in the final section of the present description.

### Example 11

### Pink eyeshadow with red and white iron oxide pigments. Formulation containing huntite compared with a formulation without huntite

Two compact powder eyeshadows having the composition indicated below were obtained by applying the procedure reported after the table.

| **Phase** | **Ingredient (INCI name)** | **Function** | **Composition of L.0 (%)** | **Composition of L.1 (%)** |
|---|---|---|---|---|
| A | Synthetic Fluorphlogopite | Filler | 62.20 | 57.80 |
| | Magnesium Myristate | Texturizer | 2.00 | 2.00 |
| | Calcium Sodium Borosilicate | Texturizer | 3.50 | 3.50 |
| | Huntite - | Texturizer, adhesion enhancer, color enhancer | - | **5.00** |
| | Boron Nitride | Texturizer | 1.50 | 1.50 |
| | Zea Mays Starch, Polyvinyl Alcohol, Glycerin | Texturizer | 2.00 | 2.00 |
| B | CI 77891 (titanium dioxide) | Pigment | 19.20 | 19.20 |
| | CI 77491 (red iron oxide) | Pigment | 0.80 | 0.80 |
| C | Polyglyceryl-10 Decamacadamiate | Binder | 0.70 | 0.70 |
| | Pentaerythrityl Tetraethylhexanoate | Binder | 2.50 | 2.50 |
| | Glyceryl Triacetyl Ricinoleate | Binder | 1.80 | 1.80 |
| | Cetearyl Ethylhexanoate | Binder | 2.70 | 2.70 |
| D | Phenoxyetanol, Caprylil glycol | Preservative | 0.90 | 0.90 |
| | Tocopherol | Antioxidant | 0.20 | 0.20 |

These eyeshadows can be prepared with a procedure similar to that of Example 4.

Experimentation on the properties of the eyeshadows obtained is presented in the final section of the present description.

### Example 12

### Nail polish compositions containing huntite.

Exemplary nail polishes according to the invention have the compositions indicated below.

| **Ingredient** | **Function** | **Composition (%)** |
|---|---|---|
| Nitrocellulose and derivatives Cellulose derivatives such as cellulose esters or ethers | Film former | 5.00-30.00 |
| Synthetic polymers and derivatives, such as acrylic copolymers, polyvinyl alcohols, polyurethanes, etc. | | |
| Silicone resins | | |
| Ether-based resins, such as polyvinyl stearyl ether | | |
| Castor oil | Plasticizer | 3.00-20.00 |
| Fatty acids and their derivatives, such as amylstearate | | |
| Citric acid derivatives, such as acetyl triethyl citrate | | |
| Derivatives of adipic and sebacic acid | | |
| Sucresters, such as sucrose benzoate | | |
| Glycol derivatives, such as glycol esters Camphor | | |
| Acetates, such as butyl acetate, isobutyl acetate | Solvent | 45.00-90.00 |
| Alcohols such as isopropyl alcohol, 1-butanol Silicone solvents such as silanes and siloxanes | | |
| Hydrocarbons such as heptane | | |
| Ketones such as acetone, methyl ethyl ketone Water | | |
| Clays and derivatives, such as bentonites and hectorites | Rheological modifier | 0.50-6.00 |
| Silicas and derivatives, such as silica dimethylsilylate | | |
| Inorganic pigments, such as iron oxides, titanium dioxide and azurites | Pigment | 1.00-20.00 |
| Organic pigments, such as carbon black, charcoal | | |
| Lakes, such as Red 6 barium lake, blue 1 aluminum lake | | |
| Natural dyes, such as carmine and betacarotene | | |
| Synthetic dyes, such as yellow 5, Red 22 Pearls, such as micas coated with iron oxides and titanium dioxide | | |
| Huntite | Texturizer, adhesion enhancer, color enhancer | 1.00-15.00 |

### Instrumental test on the ability of huntite mineral to enhance the color of pigments

To instrumentally verify the ability of the mineral to enhance the color of the pigments, a study was carried out on some simple, colored formulations, in which the color being applied was evaluated using a Konica Minolta CR-400 portable colorimeter.

The following make-up formulations were taken into consideration:
- Lipstick with Red 7 lake of Example 6
   o E.0 version without huntite and al 5% pigment
   o E.1 version with 10% huntite andq 5% pigment
   o E.2 version without huntite and 7.5% pigment
- Lipstick with red iron oxide pigment of Example 7
   o F.0 version without huntite and 5%pigment
   o F.1 version with 10% huntite and 5% pigment
   o F.2 version without huntite and 8,5% pigment
- Lipstick described in Example 1
   o A.0 version without huntite
   o A.1 version with 5% huntite
- Pearly lipstick with red and white pearl of Example 8
   o G.0 version without huntite
   o G.1 version with 10% huntite
- Dark eyeshadow with red and black iron pigments of Example 9
   o H.0 version without huntite
   o H.1 version with 5%huntite
- Brown eyeshadow with red, black and white iron oxide pigments of Example 10
   ∘ I.0 version without huntite
   ∘ I.1 version with 5% huntite
- Pink eyeshadow with red and white iron oxide pigments of Example 11
   ∘ L.0 version without huntite
   ∘ L.1 version with 5% huntite

A uniform layer of each product was spread on a white sheet, and the CIELAB coordinates (L* a* b*) were measured in order to evaluate the color intensity.

Through the CIELAB color space it is possible to give a numerical value to the perceived color through the coordinates L*, a* and b*. The coordinates a and b represent the two ranges of colors which go respectively from green to red and from blue to yellow (-a= green; +a=red; -b=blue; +b=yellow), L represents the brilliance, then the white value (-L=black; +L=white).

### Test on lipsticks E and F of Examples 6 and 7

Lipsticks E and F were prepared according to the following scheme: a reference lipstick, without huntite; a lipstick like the previous one, but with huntite which enhances the color rendering; a lipstick without huntite but with an increase in pigment in order to obtain a color rendering similar to the version with huntite.

Since the prevailing color in each formulation is red, the value a* was compared, which according to the CIELAB method is indicative of the intensity of red. Therefore, the more intense the color of the lipstick, the **higher** the a* value will be.

The results are set out in the following Table 1, and represented in the form of a histogram in **Figure 9** of the accompanying drawings.

**TABLE 1**

| **Lipstick E** | | | | **Lipstick F** | | | |
|---|---|---|---|---|---|---|---|
| | **E.1** | **E.2** | **E.0** | | **F.1** | **F.2** | **F.0** |
| **a*** | 56.46 | 55.91 | 51.45 | **a*** | 40.11 | 40.41 | 36.86 |

As can be seen from the diagrams, the lipsticks containing huntite have a higher a* value, which measures the intensity of the red, than in the reference versions without huntite. Lipsticks A.2 and B.2, which contain a greater quantity of pigment, demonstrate that the use of huntite allows to considerably reduce the quantity of pigment, maintaining the same color rendering.

### Test on lipsticks A and G of Examples 1 and 8

The lipsticks A and G of Examples 1 and 8 were prepared in versions with and without huntite.

Since the prevailing color in each formulation is red, the value a* was compared, which according to the CIELAB method is indicative of the intensity of red. Therefore, the more intense the color of the lipstick, the **higher** the a* value will be.

The results are set out in the following Table 2, and represented in histogram form in **Figure 10** of the accompanying drawings.

**TABLE 2**

| **Lipstick A** | | | **Lipstick G** | | |
|---|---|---|---|---|---|
| | **A.1** | **A.0** | | **G.1** | **G.0** |
| **a*** | 52.77 | 48.06 | **a*** | 35.62 | 26.16 |

As can be seen from the diagrams, the lipsticks containing huntite have a higher a* value, which measures the intensity of red, than in the reference versions without huntite.

### Test on eyeshadows H, I and L of Examples 9, 10 and 11

The eyeshadows H, I and L of Examples 9, 10 and 11 were prepared in versions with and without huntite.

Since there is not a single prevailing color in these formulas, the L* value was compared, which according to the CIELAB method is indicative of the intensity of white. Therefore, the more intense the color of the eyeshadow, therefore not dirty by white, the **lower** the L* value will be.

The results are set out in the following Table 3, and represented in histogram form in **Figure 11** of the accompanying drawings.

**TABLE 3**

| **Eyeshadow H** | | | **Eyeshadow I** | | | **Eyeshadow L** | | |
|---|---|---|---|---|---|---|---|---|
| | **H.1** | **H.0** | | **I.1** | **I.0** | | **L.1** | **L.0** |
| **L*** | 35.82 | 42.16 | **L*** | 45 | 48.28 | **L*** | 75.57 | 78.56 |

As can be seen from the diagrams, the eyeshadows containing huntite have a lower L* value, which measures the intensity of white, than in the reference versions. It can be deduced that they are darker, therefore the rendering of the colors is improved.

## Claims

1. Use of a natural huntite powder as a color enhancer for pigments, with possible additional functions, in cosmetic products for make-up, wherein said cosmetic products contain one or more pigments, and from 2% to 30% by weight of powdered huntite, and
wherein the color of said cosmetic products, evaluated using a Konica Minolta CR-400 portable color meter and using the CIELAB coordinates (L* a* b*), is more intense than the color of cosmetic products having the same composition without huntite.

2. Use of huntite powder according to claim 1, wherein said huntite powder has one or more of the following additional functions: texturizing agent, filler, mattifying agent, dispersant, adhesion enhancer, soft-focus effect enhancer.

3. Use of huntite powder according to claims 1 or 2, wherein said huntite powder has a particle size comprised between 0.1 µm and 50 µm.

4. Use of huntite powder according to claim 3, wherein said huntite powder has a particle size comprised between 0.5 µm and 10 µm.

5. Use of huntite powder according to claim 4, wherein said huntite powder is a submicrometric powder.

6. A cosmetic composition for make-up containing one or more pigments, and from 2% to 30% by weight of a powdered color enhancer, wherein said color enhancer is a natural huntite powder, and
wherein the color of said cosmetic products, evaluated using a Konica Minolta CR-400 portable color meter and using the CIELAB coordinates (L* a* b*), is more intense than the color of cosmetic products having the same composition without huntite.

7. The cosmetic composition according to claim 6, wherein said huntite has one or more of the following additional functions: texturizing agent, filler, mattifying agent, dispersant, adhesion enhancer, soft-focus effect enhancer.

8. The cosmetic composition according to claims 6 or 7, wherein the product made from the composition is a lipstick or a concealer, and the composition comprises from 3% to 12% by weight of huntite powder as a color enhancer, with additional functions of texturizer and adhesion enhancer.

9. The cosmetic composition according to claims 6 or 7, wherein the product made from the composition is an eye shadow compact powder, and the composition comprises 2 % to 15% by weight of huntite powder as color enhancer, with additional functions of texturizer and adhesion enhancer.

10. The cosmetic composition according to claims 6 or 7, wherein the product made from the composition is a nail polish, and the composition comprises from 2 % to 15% by weight of huntite powder as a color enhancer.

11. The cosmetic composition according to claims 6 or 7, wherein the product made from the composition is an opacifying compact powder, and the composition comprises from 20% to 30% by weight of huntite powder as a white pigment, texturizing agent and soft-focus enhancer.

## Patentansprüche

1. Verwendung eines natürlichen Huntitpulvers als Farbverstärker für Pigmente, mit möglichen zusätzlichen Funktionen, in kosmetischen Produkten für Make-up, wobei die kosmetischen Produkte ein oder mehrere Pigmente und 2 bis 30 Gew.-% pulverisierten Huntit enthalten, und wobei die Farbe der kosmetischen Produkte, bewertet mit einem tragbaren Farbmessgerät Konica Minolta CR-400 und unter Verwendung der CIELAB-Koordinaten (L* a* b*), intensiver ist als die Farbe der kosmetischen Produkte mit derselben Zusammensetzung ohne Huntit.

2. Verwendung von Huntitpulver nach Anspruch 1, wobei das Huntitpulver eine oder mehrere der folgenden zusätzlichen Funktionen aufweist: Texturierungsmittel, Füllstoff, Mattierungsmittel, Dispergiermittel, Haftungsverstärker, Verstärker des Weichzeichnereffekts.

3. Verwendung von Huntitpulver nach Anspruch 1 oder 2, wobei das Huntitpulver eine Teilchengröße zwischen 0,1 µm und 50 µm aufweist.

4. Verwendung von Huntitpulver nach Anspruch 3, wobei das Huntitpulver eine Teilchengröße zwischen 0,5 µm und 10 µm aufweist.

5. Verwendung von Huntitpulver nach Anspruch 4, wobei das Huntitpulver ein submikrometrisches Pulver ist.

6. Kosmetische Zusammensetzung zum Schminken, die ein oder mehrere Pigmente und 2 bis 30 Gew.-% eines pulverförmigen Farbverstärkers enthält, wobei der Farbverstärker ein natürliches Huntitpulver ist, und wobei die Farbe der kosmetischen Produkte, bewertet mit einem tragbaren Farbmessgerät Konica Minolta CR-400 und unter Verwendung der CIELAB-Koordinaten (L* a* b*), intensiver ist als die Farbe der kosmetischen Produkte mit derselben Zusammensetzung ohne Huntit.

7. Kosmetische Zusammensetzung nach Anspruch 6, wobei das Huntit eine oder mehrere der folgenden zusätzlichen Funktionen hat: Texturierungsmittel, Füllstoff, Mattierungsmittel, Dispergiermittel, Haftungsverstärker, Verstärker des Weichzeichnereffekts.

8. Kosmetische Zusammensetzung nach Anspruch 6 oder 7, wobei das aus der Zusammensetzung hergestellte Produkt ein Lippenstift oder ein Concealer ist und die Zusammensetzung 3 bis 12 Gew.-% Huntitpulver als Farbverstärker mit zusätzlichen Funktionen als Texturgeber und Haftungsverstärker enthält.

9. Kosmetische Zusammensetzung nach Anspruch 6 oder 7, wobei das aus der Zusammensetzung hergestellte Produkt ein Lidschatten-Kompaktpuder ist und die Zusammensetzung 2 bis 15 Gew.-% Huntit-Puder als Farbverstärker mit den zusätzlichen Funktionen eines Texturgebers und Haftungsverstärkers enthält.

10. Kosmetische Zusammensetzung nach Anspruch 6 oder 7, wobei das aus der Zusammensetzung hergestellte Produkt ein Nagellack ist und die Zusammensetzung 2 bis 15 Gew.-% Huntitpulver als Farbverstärker enthält.

11. Kosmetische Zusammensetzung nach Anspruch 6 oder 7, wobei das aus der Zusammensetzung hergestellte Produkt ein opakisierender Kompaktpuder ist und die Zusammensetzung 20 bis 30 Gew.-% Huntitpulver als Weißpigment, Texturierungsmittel und Weichzeichner enthält.

## Revendications

1. Utilisation d'une poudre de huntite naturelle comme rehausseur de couleur pour les pigments, avec d'éventuelles fonctions supplémentaires, dans des produits cosmétiques pour le maquillage, dans lesquels lesdits produits cosmétiques contiennent un ou plusieurs pigments, et de 2% à 30% en poids de huntite en poudre, et dans lequel la couleur desdits produits cosmétiques, évaluée à l'aide d'un colorimètre portable Konica Minolta CR-400 et en utilisant les coordonnées CIELAB (L* a* b*), est plus intense que la couleur des produits cosmétiques ayant la même composition sans huntite.

2. Utilisation de poudre de huntite selon la revendication 1, dans laquelle ladite poudre de huntite a une ou plusieurs des fonctions supplémentaires suivantes: agent texturant, charge, agent matifiant, dispersant, améliorant l'adhérence, améliorant l'effet soft-focus.

3. Utilisation de poudre de huntite selon les revendications 1 ou 2, dans laquelle ladite poudre de huntite a une taille de particule comprise entre 0,1 µm et 50 µm.

4. Utilisation de poudre de huntite selon la revendication 3, dans laquelle ladite poudre de huntite a une taille de particule comprise entre 0,5 µm et 10 µm.

5. Utilisation de poudre de huntite selon la revendication 4, dans laquelle ladite poudre de huntite est une poudre submicrométrique.

6. Composition cosmétique pour le maquillage contenant un ou plusieurs pigments, et de 2% à 30% en poids d'un rehausseur de couleur en poudre, dans laquelle ledit rehausseur de couleur est une poudre de huntite naturelle, et dans lequel la couleur desdits produits cosmétiques, évaluée à l'aide d'un colorimètre portable Konica Minolta CR-400 et en utilisant les coordonnées CIELAB (L* a* b*), est plus intense que la couleur des produits cosmétiques ayant la même composition sans huntite.

7. Composition cosmétique selon la revendication 6, dans laquelle ladite huntite a une ou plusieurs des fonctions supplémentaires suivantes: agent texturant, agent de remplissage, agent matifiant, dispersant, renforçant l'adhérence, renforçant l'effet soft-focus.

8. Composition cosmétique selon les revendications 6 ou 7, dans laquelle le produit fabriqué à partir de la composition est un rouge à lèvres ou un correcteur, et la composition comprend de 3% à 12% en poids de poudre de huntite en tant que rehausseur de couleur, avec des fonctions supplémentaires de texturant et de rehausseur d'adhérence.

9. Composition cosmétique selon les revendications 6 ou 7, dans laquelle le produit fabriqué à partir de la composition est une poudre compacte pour fard à paupières, et la composition comprend de 2% à 15% en poids de poudre de huntite comme rehausseur de couleur, avec des fonctions supplémentaires de texturant et de rehausseur d'adhérence.

10. Composition cosmétique selon les revendications 6 ou 7, dans laquelle le produit fabriqué à partir de la composition est un vernis à ongles, et la composition comprend de 2% à 15% en poids de poudre de huntite en tant que rehausseur de couleur.

11. Composition cosmétique selon les revendications 6 ou 7, dans laquelle le produit fabriqué à partir de la composition est une poudre compacte opacifiante, et la composition comprend de 20% à 30% en poids de poudre de huntite en tant que pigment blanc, agent texturant et améliorateur de soft-focus.
